# EUROPEAN PATENT APPLICATION

(11) **EP 1 551 104 A2**
(43) Date of publication of application: **06.07.2005**
(21) Application number: 05250015.4
(22) Date of filing: 05.01.2005
(51) Int. Cl.: H04B 1/08

(54) **Front-part detachable car stereo**

(30) Priority: 05.01.2004 AR 0400010; 03.01.2005 AR 0500001
(71) Applicant: Famar Fueguina S.A., Tierra del Fuego (AR)
(72) Inventor: Abolsky, Gabriel, Tierra del Fuego (AR)
(74) Representative: Roberts, Mark Peter

(57) **Abstract**

A car-stereo with a detachable front part wherein appart from having part of the command devices located at said detachable front, the novelty is based on the fact that said detachable front is a portable player of compressed formats in digital files, with its corresponding screen, as well as a radio tuner which in its rear part has a series of contacts that may be coupled with corresponding contacts located at the front of said stereo, wherein said device tightly fits in a docking station on the front of the car-stereo.

## Description

This invention, which is additional to the Argentine Patent Application No. P04 01 00010, is directed to a car-stereo with a detachable front part having security elements. More precisely, this invention refers to a stereo which detachable front part is a device having commands to play the stereo where said commands are connected by means of electric contacts located at the fixed front thereof.

Usually, car-stereos have a detachable front, which has no independent function with regard to the device commands, since when said detachable front is detached from the device, it does not have any kind of independent function.

Nowadays, it is usual that detachable fronts that have all the device commands also have a series of mechanical contacts and links to the fixed front thereof, said detachable fronts being of a small size so that their users may easily carry them once detached.

An exception to the above-described stereo is disclosed in US Patent No. 5,537,673, which detachable front is a cellular phone.

It is an object of this invention to achieve that a portable player device with digital memory be the front of the stereo where the own functions of both devices and the security provided by the detachable front are combined.

It is the main object of this invention to provide a car-stereo with a detachable front in which appart from the fact that part of the command devices are located at said detachable front, the novelty is based on a detachable front which is a portable player device with compressed formats in digital files as well as a radio tuner, which at its rear part has a series of contacts that may be coupled with corresponding contacts in the front of the stereo.

In a preferred embodiment of this invention, said device remains tightly fixed when fitted in the front of the stereo.

In said preferred embodiment of the invention, the device screen is the screen which indicates the radio sintony of the car.

The main object of this invention as well as its advantages may be seen in the following description of one preferred embodiment of the invention, with reference to the drawings, in which:
Figure 1 is a front view of the set;
Figure 2 is a front view of the setereo with a detached front;
Figure 3 is a rear view of the detachable device;
Figure 4 is a scheme of the device connections to the stereo.

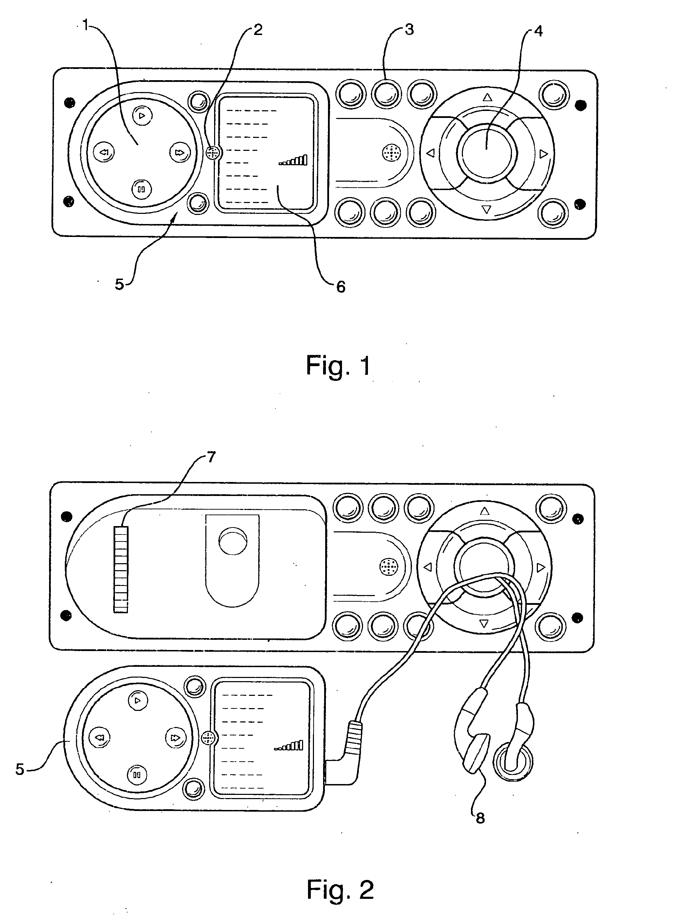

In Figure 1, the front of the stereo with the detachable device 5 fitted in it is shown.

The detachable device 5 has the screen 6, which, on the one hand, indicates the sintony of the device when it has been detached and, on the other, it indicates the sintony of the car-stereo.

Likewise, the radio has its own control keys 3 and 4.

When the device 5 is detached, it has a button 1 for the sintony tuning as well as a microphone 2 to record information thereon.

In figure 2, a detached device 5 is shown having the corresponding headphones 8 connected thereto and it may be seen that the device is fitted in a docking station having a series of contacts 7 inside it.

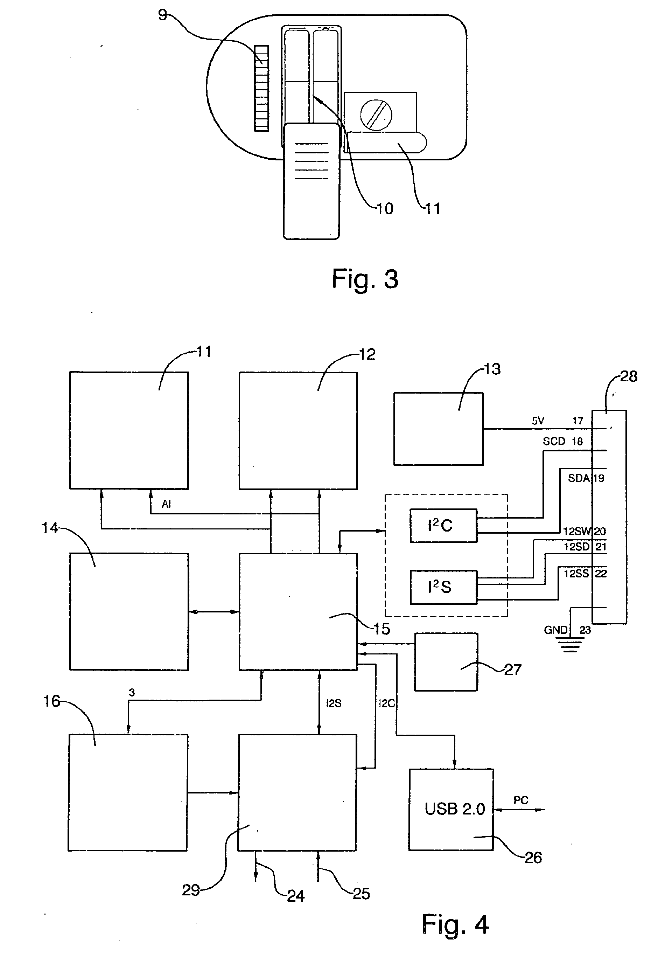

When the device is fitted, the contacts 7 are connected with a series of contacts 9 located at the rear part of the device 5, as shown in Figure 3.

Likewise, at the rear of the device 5 shown in Figure 3, there is a compartment 10 housing the batteries that enable that said device works in an antonomous way and a fixing element to fasten it to the device supporting element.

In figure 4, which is scheme of the circuit of the detachable device 5, it may be seen that it is comprised by a screen 11 which receives the signals and is parallely connected with the flash memory 12 from the digital signal processor 15.

Likewise, the processor 15 is connected to a card slot 14 with which information holders, such as cards containing information that may be read or recorded therefrom, may be connected.

The processor 15 is also interconnected with the FM 16 tuner which has only one chip and an output connected with the audio codifyier/decodifyier 29, which has an outlet for headphones 24 and a microphone inlet 25, where the audio codifyier/decodifyier 29 is interconnected with the processor 15.

The processor 15 is also connected with a USB 26-type connection in order to connect it with a PC and keep in or send sound from the device.

A detachable kewboard may be connected to the processor 15 in order to send it signals and control the different functions of the processor.

The processor 15 is connected with the I²C interphase bus which outlets, SCD 18 and SDA 19, are connected by sending information to the connector 28 as well as outlets 12SW 20, 12SD 21 and 12SS 22 of the I²S interphase sound bus receiving the signals in an audio compressed format from processor 15.

A 5V power supply is connected by line 17 to the connector 28 while the connection 23 is connected to ground.

The above-mentioned connector 28 has contacts 9 and is located at the rear part of the device 5.

Therefore, when the device 5 is fitted, the processor 15 contacts the vehicle radio in order to show in the screen what the user communicates to the radio through its own buttons and it may also show what is kept in the files of the card slot 14.

When the device 5 is detached, the power supply is obtained from the battery in the power source 13, and the processor 15, with the complete control of the device, enables the user to listen by means of the headphones 8 to the radio station tuned by means of the tuner 16 or listen to what is recorded in the card inserted into the slot 14, said headphones 8 being connected with the outlet 24 of the audio codifyier/decodifyier 29.

## Claims

1. A car-stereo with a detachable front wherein, appart from the fact that its command devices are located at said detachable front, it is **characterized in that** said detachable front is a portable player of compressed formats in digital files, with a screen as well as a radio tuner which in its rear part has a series of contacts that may be coupled with corresponding contacts located at the front of said radio, wherein said device tighlty fits in a docking station at the front of said vehicle stereo.

2. A car-stereo with a detachable front in accordance with claim 1 **characterized in that** said device remains tightly fit when fitted in the front of said stereo.

3. A car-stereo with a detachable front in accordance with claim 1, **characterized in that** said device screen is a screen that indicates the sintony of the car-stereo.
